# FASCICULE DE BREVET EUROPEEN

(11) **EP 1 741 387 B1**
(45) Date de publication et mention de la délivrance du brevet: **03.06.2009**
(21) Numéro de dépôt: 06291002.1
(22) Date de dépôt: 21.06.2006
(51) Int. Cl.: A61B 5/08, A61B 5/11

(54) **Appareillage pour la détection non invasive de la survenue d'apnées ou d'hypopnées chez un patient**
Vorrichtung zur nicht invasiven Detektion von Schlafapnoe oder Hypopneen
Device for non invasive detection of occurence of sleep apnea or hypopnea

(30) Priorité: 05.07.2005 FR 0507121; 20.04.2006 FR 0603484
(43) Date de publication de la demande: 10.01.2007
(73) Titulaire: ELA MEDICAL, 92541 Montrouge (FR)
(72) Inventeur: Vincent, Elodie, 92330 Sceaux (FR); Vitali, Luca, 10019 Strambino (IT)
(74) Mandataire: Dupuis-Latour, Dominique

(56) Documents cités:
- EP-A- 1 336 422
- EP-A- 1 413 330
- EP-A- 1 433 496
- EP-A- 1 533 001
- EP-A- 1 537 894
- EP-A- 1 674 035
- US-A1- 2004 111 038
- BOMBARDINI T ET AL: "OPERATOR INDEPENDENT LEFT VENTRICULAR FUNCTION MONITORING DURING PHARMACOLOGICAL STRESS ECHO WITH THE NEW PEAK TRANSCUTANEOUS ACCELERATION SIGNAL" CANADIAN JOURNAL OF INFORMATION SCIENCE - REVUE CANADIENNE DES SCIENCES DE L'INFORMATION, TORONTO, CA, vol. 85, no. 3, mars 2001 (2001-03), pages 286-289, XP008018630 ISSN: 0380-9218

## Description

L'invention concerne un appareillage pour la détection non invasive de la survenue d'apnées ou d'hypopnées chez un patient.

De façon générale, la pathologie respiratoire connue sous le nom de "syndrome d'apnée du sommeil" (SAS) est caractérisée par la survenue fréquente (au moins 10 à 20 fois par heure) d'apnées pendant une phase de sommeil du patient, une "apnée" (ou pause respiratoire) étant définie comme un arrêt temporaire de la fonction respiratoire de durée supérieure à 10 secondes. Elle peut également être caractérisée par la survenue d'hypopnées dans les mêmes conditions, une "hypopnée" étant définie comme une décroissance importante (mais sans interruption) du débit respiratoire, typiquement une décroissance de plus de 50 % par rapport à une moyenne de référence antérieure.

Face à cette pathologie, qui atteint plus de 4% de la population et plus de 50% des patients souffrant d'insuffisance cardiaque, le système nerveux autonome s'adapte mais avec un effet délétère sur le sommeil, l'interruption ou la réduction du débit respiratoire entraînant une diminution de la concentration en oxygène du sang ainsi que des micro-réveils inconscients. Il s'ensuit en phase d'éveil une somnolence diurne avec perte d'attention et un accroissement des risques d'accident de la route. Par ailleurs, la réponse adaptative physiologique, puis pathologique, de certains organes dont le coeur et l'appareil circulatoire entraîne une plus grande incidence de troubles tels qu'hypertension artérielle, arythmies ventriculaires, infarctus myocardique et insuffisance cardiaque.

On connaît diverses techniques pour détecter de tels troubles respiratoires du sommeil.

Ainsi, le EP-A-0 970 713 (ELA Medical) divulgue un dispositif diagnostiquant la survenue d'une apnée à partir du signal de ventilation-minute (signal VE, dit également signal MV), paramètre à prépondérance physiologique généralement obtenu par une mesure d'impédance transthoracique donnant en continu une indication du rythme respiratoire du patient.

Un inconvénient de cette technique tient au fait qu'elle présuppose que le patient soit appareillé d'un implant, ce qui en limite très strictement son utilité et son efficacité.

La présente invention propose une autre approche du diagnostic des pathologies de l'activité respiratoire, au moyen d'un appareillage externe, donc non invasif, qui puisse être mis en place aisément sur le patient, éventuellement par le patient lui-même, et qui n'introduise aucune gêne comparable à celle de certains appareils mettant en oeuvre des masques respiratoires devant être portés pendant le sommeil.

Un des buts de la présente invention est de mettre à la disposition des praticiens un tel appareillage qui, d'une part, soit simple à mettre en oeuvre et non invasif (et donc applicable à tout patient, qu'il soit ou non muni d'un implant) et qui, d'autre part, délivre au praticien non seulement des données brutes, mais également une aide au diagnostic, notamment à partir des critères ayant abouti au diagnostic des apnées ou hypopnées.

Essentiellement, l'invention propose une détection des apnées ou hypopnées à partir d'une mesure de l'accélération endocardiaque, plus précisément basée sur l'analyse des pics d'accélération endocardiaque, qui est un paramètre reflétant de façon non artefactée et avec un très faible temps de réponse les variations de la contractilité du myocarde.

On connaît, par exemple d'après le EP-A-1 413 330 ou le EP-A-1 433 496 (ELA Medical) un dispositif comprenant des moyens de mesure de l'accélération endocardiaque, et capable de diagnostiquer et de traiter des troubles respiratoires.

Mais, il s'agit d'un implant de type stimulateur cardiaque, qui ne peut donc être mis en oeuvre que dans des situations particulières et pour une population de patients limitée.

Par ailleurs, Bombardini et all. "Operator-independent left ventricular function monitoring during pharmacological stress echo with the new peak transcutaneous acceleration signal," Canadian Journal of Information Science, Toronto, CA, Vol. 85, n°3, mars 2001, pp. 286-289, ont montré la faisabilité de la mesure du PEA par un capteur accélérométrique cutané, pour évaluer les variations de ce paramètre dans le cadre d'une étude pharmacologique.

L'invention propose de combiner les approches présentées dans ces divers documents, pour permettre la détection non invasine des troubles respiratoires du sommeil par l'analyse d'un signal PEA.

L'appareillage de l'invention comprend des moyens de détection des apnées ou hypopnées tels que dévulgués par le EP-A-1 413 330 précité correspondant au préambule de la revendication 1. La partie caractérisante de cette revendication 1 énonce les éléments particuliers de l'invention. Les sous-revendications visent des formes de mise en oeuvre spécifiques.

On va maintenant décrire un exemple de réalisation de l'invention, en référence aux dessins annexés.

La figure 1 est une vue schématique de l'appareillage de l'invention, relié à un patient allongé.

La figure 2 précise la structure interne du capteur accélérométrique utilisé. La figure 3 est un schéma des circuits d'entrée et de mise en forme du signal délivré par le capteur de la figure 2.

La figure 4 est un chronogramme montrant les variations au cours de trois cycles cardiaques successifs de l'accélération endocavitaire ainsi que de l'électrocardiogramme de surface correspondant.

La figure 5 illustre les variations du rapport entre moyenne à court terme et moyenne à long terme de l'accélération endocavitaire au cours d'un épisode d'apnée typique.

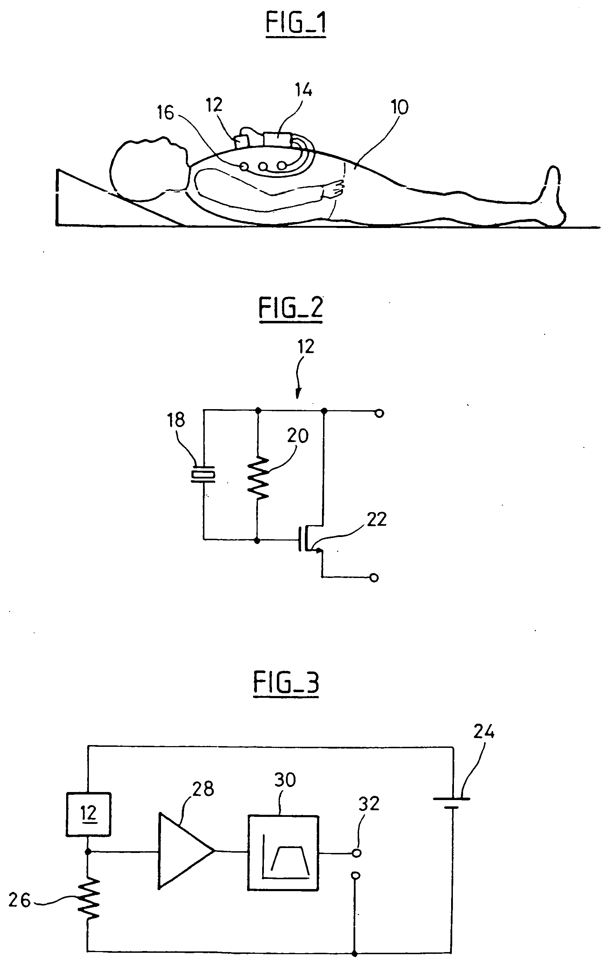

Sur la figure 1, la référence 10 désigne un patient allongé, sur lequel on souhaite diagnostiquer les troubles du sommeil. De façon caractéristique de l'invention, le patient est équipé d'un capteur accélérométrique externe 12 placé dans la région du sternum et maintenu plaqué contre la paroi thoracique, par exemple (mais de manière non limitative) par un "patch" tel que celui utilisé pour le maintien des électrodes ECG, le tout étant éventuellement recouvert d'un bandage adhésif. Comme on peut le voir, la mise en oeuvre de l'appareillage de l'invention est particulièrement simple et non invasive, dans la mesure où il suffit d'appliquer le capteur accélérométrique sur la cage thoracique du patient. Et, pour ce dernier, elle n'implique pas d'autre gêne que le port d'un enregistreur Holter, technique couramment utilisée pour d'autres types de diagnostics ambulatoire.

Le capteur 12 est relié à un dispositif enregistreur 14, par exemple un enregistreur Holter capable d'enregistrer sur une longue durée (au moins celle d'une période de sommeil) les signaux délivrés par le capteur, et de traiter et convertir ceux-ci pour en dériver les informations nécessaires au diagnostic des apnées ou hypopnées.

Le patient peut être également équipé d'électrodes 16 de recueil d'un ECG de surface, également reliées à l'enregistreur 14, permettant en particulier d'obtenir un signal de fréquence cardiaque avec lequel pourra être croisée l'information dérivée du signal délivré par le capteur accélérométrique 12.

La figure 2 montre plus en détail la structure du capteur accélérométrique 12, qui comporte un élément sensible piézoélectrique 18 polarisé par une résistance 20 et associé à un transistor MOS 22 de préamplification. Comme on peut le voir sur la figure 3, le capteur 12 est polarisé par une source de tension 24 en série avec une résistance 26. Le signal de sortie du capteur est amplifié par un amplificateur 28 puis mis en forme par un filtre passe-bande 30 et délivré à la sortie 32 pour numérisation et traitement ultérieurs.

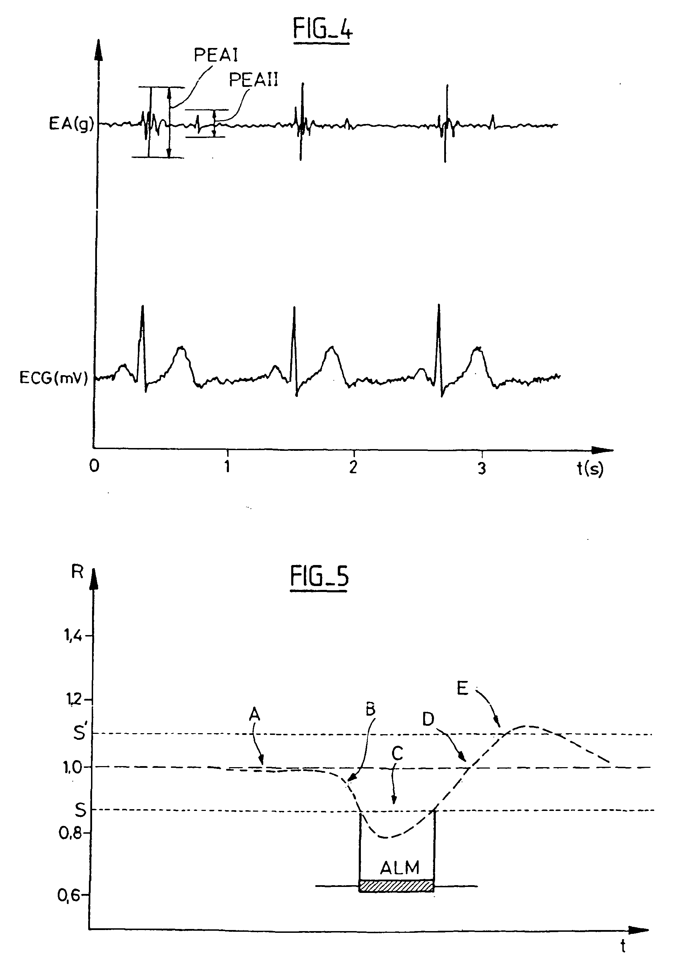

Sur la figure 4 on a représenté (courbe du haut), les variations de l'accélération endocardiaque (EA) mesurée par le capteur 12. On a également illustré sur cette figure le tracé d'un électrocardiogramme de surface (ECG) correspondant, au cours de trois cycles cardiaques consécutifs.

Le signal d'accélération endocardiaque mesuré au cours d'un cycle cardiaque forme, entre autres, deux pics correspondant aux deux bruits majeurs qu'il est possible de reconnaître dans chaque cycle d'un coeur sain. Le EP-A-0 655 260 (Sorin Biomedica Cardio SpA) décrit une manière de traiter un signal d'accélération endocavitaire (EA) pour en dériver, notamment, ces deux valeurs de pic d'accélération endocardiaque (PEA), utiles en particulier pour la détection de troubles cardiaques et le déclenchement ou non d'une thérapie de défibrillation.

Par "pic" on entendra la valeur maximale crête-à-crête du signal d'accélération séparant les deux extrema, positif et négatif, correspondant aux écarts PEA I et PEA II indiqués sur le chronogramme de la figure 4.

Plus précisément, le premier pic d'accélération endocardiaque ("PEA I") correspond à la fermeture des valvules mitrale et tricuspide, au début de la phase de contraction ventriculaire isovolumique (systole). Les variations de ce premier pic sont étroitement liés aux variations de la pression dans le ventricule (l'amplitude du pic PEA I étant, plus précisément, corrélée au maximum positif de la variation de pression dP/dt dans le ventricule gauche) et peuvent donc constituer un paramètre représentatif de la contractilité du myocarde, elle-même liée au niveau d'activité du système sympathique.

Le second pic d'accélération endocardiaque ("PEA II"), quant à lui, correspond à la fermeture des valvules aortique et pulmonaire, au moment de la phase de relaxation ventriculaire isovolumique. Ce second pic, qui est produit par la décélération brusque de la masse sanguine en mouvement dans l'aorte, constitue un paramètre représentatif de la pression sanguine périphérique au début de la diastole. Il constitue également un paramètre-clé du processus physiologique conduisant à la survenue d'une syncope vasovagale.

Les valeurs de pics PEA I et/ou PEA II sont relevées au cours de cycles successifs, ainsi qu'éventuellement la fréquence cardiaque.

Ces signaux peuvent être traités de diverses manières.

Une première technique consiste à déterminer, cycle à cycle, les valeurs absolues que prennent ces paramètres et à fixer des seuils de déclenchement d'alarme ― ou, de préférence, déterminer une valeur moyennée de ces paramètres sur un nombre prédéterminé de cycles, pour éviter les influences de la variabilité cycle-à-cycle (dispersion des mesures) et celles des évènements brefs non significatifs.

Pour diagnostiquer la présence ou non d'une apnée ou hypopnée, un ou plusieurs seuils sont fixés, et chacun des paramètres PEA I ou PEA II (ou une combinaison de ces deux paramètres) est comparé à un seuil prédéterminé. Le résultat de la comparaison peut être combiné de diverses manières avec le résultat de comparaisons semblables d'autres paramètres (notamment la fréquence cardiaque) pour produire un signal de sortie à deux états, l'un des états étant associé à une situation normale et l'autre état étant associé à une alerte d'apnée ou hypopnée.

Pour améliorer la spécificité de la détection, et notamment pour tenir compte des différences de valeur de base des paramètres PEA d'un individu à l'autre, il peut être avantageux d'analyser les variations de ces paramètres plutôt que leurs valeurs absolues.

Une manière de procéder consiste à analyser la différence entre une moyenne à court terme et une moyenne à long terme du même paramètre. Si ce paramètre varie peu, la différence sera faible et les deux valeurs finiront par coïncider. En revanche, dès que le paramètre deviendra instable, la moyenne à court terme va suivre les variations du paramètre plus rapidement que la moyenne à long terme. La différence entre les deux moyennes ne sera plus nulle ou quasi-nulle, mais prendra une valeur positive (en cas d'augmentation du paramètre) ou négative (en cas de diminution), la valeur absolue de cet écart dépendant du paramètre analysé et de la vitesse de variation de celui-ci.

Il est également possible de suivre non pas la différence, mais le ratio entre moyenne à court terme et moyenne à long terme des paramètres PEA I et/ou PEA II.

Un exemple d'algorithme de détection consiste à calculer une moyenne mobile à court terme des valeurs cycle à cycle du paramètre PEA I, par exemple sur quatre cycles consécutifs. Simultanément, une moyenne mobile à long terme, par exemple sur 500 cycles, de ce même paramètre est calculée et mise à jour. Les deux moyennes sont alors comparées pour donner un ratio R, où R = moyenne à court terme/moyenne à long terme.

Les variations de ce ratio sont illustrées sur la figure 5 : en régime stationnaire, le ratio R présente une valeur à peu près constante et voisine de l'unité ; en revanche, lors d'un épisode d'apnée, la diminution de la saturation en oxygène dans le sang induit une réduction de la contractilité cardiaque qui se traduit par une diminution correspondante de l'amplitude du paramètre PEA I pendant plusieurs battements consécutifs, et donc une diminution corrélative de la valeur de R. Cette situation est illustrée en A sur la figure 5, où est indiqué le début de l'épisode d'apnée, et en B pour le moment où l'accélération endocardiaque chute brutalement suite à la réduction de la contractilité.

Il est possible de déclencher une alarme en définissant pour le ratio R un seuil approprié S, par exemple S = 0,85 ou S = 0,9, et en comparant la valeur courante du ratio R à ce seuil. Chaque fois que la ratio R tombe en dessous de ce seuil (situation illustrée en C sur la figure 5), une alarme (ALM) est déclenchée.

Cette alarme peut être déclenchée immédiatement au moment du franchissement du seuil, ou bien de manière conditionnelle, par exemple seulement si le ratio R descend sous le seuil pendant une durée minimale prédéterminée (par exemple 5 secondes) ou un nombre de cycles prédéterminé (par exemple 5 cycles).

L'activation de l'alarme indique ainsi un épisode de dépression soudaine de la contractilité cardiaque, qui est associé à un épisode d'apnée ou hypopnée. Ceci déclenche en réponse l'inscription dans une mémoire de l'enregistreur 14d'informations de diagnostic telles que marqueur de survenue d'une apnée, horodatage de cette apnée, durée de l'alarme, ... Il est également possible d'y enregistrer simultanément les signaux d'accélération endocardiaque et/ou les valeurs des différents paramètres PEA. Cette mémorisation peut être éventuellement déclenchée selon certaines conditions programmées ou sur détection de certains états (sommeil confirmé) ou évènements (survenue d'un épisode apnéique). Les signaux mémorisés pourront être ultérieurement visualisés par un praticien équipé d'un programmateur permettant de lire le contenu de la mémoire de l'implant. II sera également possible, à ce stade, d'analyser le signal PEA afin d'en extraire certains paramètres qui ne sont pas déterminables en temps réel, tels que la variabilité sinusale, ou pour simuler l'application d'algorithmes de détection afin de choisir les plus approprié au cas du patient.

Par ailleurs on observe généralement après la fin de l'épisode d'apnée un phénomène de rebond, correspondant à une augmentation transitoire de la contractilité cardiaque (ce rebond est illustré en E sur la figure 2). Plus précisément, à la fin d'un épisode d'apnée survient souvent un micro-réveil au cours duquel le système sympathique est activé en réaction aux évènements antérieurs ; cette réaction prend fin spontanément après un certain nombre de cycles cardiaques, le ratio R retournant progressivement à sa valeur de base voisine de l'unité.

Ce phénomène de rebond peut être détecté et utilisé pour confirmer la survenue de l'épisode d'apnée. À cet effet, on définit un second seuil S' de valeur supérieure à l'unité, par exemple S' = 1,1 et on détecte le franchissement de ce second seuil S' après détection d'un épisode d'apnée. On notera que le franchissement du seuil S' révèle seulement une réaction positive du système sympathico-vagal à l'état anormal induit par l'apnée, réaction qui n'est pas en soi pathologique et ne doit pas déclencher d'alarme et ni de thérapie particulière.

En revanche, la détection conjointe (i) d'une diminution du PEA révélant une réduction de la contractilité, détectée par le franchissement du premier seuil S (ratio R < 0,85), suivie (ii) d'une augmentation du PEA, détectée par le franchissement du second seuil (ratio R > 1,1), et ce (iii) dans un intervalle de temps prédéterminé, par exemple dans un laps de temps d'une minute, est un indice fort d'un état non physiologique rencontré typiquement chez des patients souffrant de troubles du sommeil.

La détection d'un tel profil typique de variation du PEA peut être utilisée pour calculer un indice spécifique, permettant de diagnostiquer chez le sujet la présence ou non d'une instabilité du système sympathico-vagal.

En variante ou en complément du paramètre PEA I, il est possible de prendre en compte le PEA II comme dans l'exemple qui précède. Le PEA II peut être utilisé comme indicateur d'une contractilité réduite, dans la mesure où il est corrélé aux variations de la pression sanguine. Plus précisément, une contractilité réduite combinée à un ralentissement du rythme cardiaque provoque une diminution de la pression sanguine, qui se traduit par une réduction de l'amplitude du PEA II. L'analyse de ce paramètre permet ainsi de confirmer les épisodes d'apnée.

D'autres types d'analyses, plus complexes, peuvent également être mises en oeuvre pour améliorer encore la qualité du procédé de détection, par exemple des techniques de corrélation, d'analyse de morphologie du signal, d'analyse fréquentielle, d'analyse par ondelettes, d'analyse en composantes principales, etc.

Il est également possible d'utiliser un processus de type "machine à états", où les résultats des comparaisons aux divers seuils sont appliqués à un système à transition d'états et à mémoire, qui prend la décision de déclencher une alerte d'apnée ou hypopnée en fonction d'un schéma d'évolution plus complexe.

On peut en outre prévoir que le système soit auto-adaptatif, c'est-à-dire qu'il puisse s'adapter à des variations sur le long terme, afin d'améliorer ultérieurement la spécificité du système de détection.

Enfin, pour améliorer la sensibilité et la spécificité de la détection des épisodes d'apnée ou d'hypopnée, il est possible de combiner l'analyse du PEA à une analyse parallèle de la fréquence cardiaque. En effet, cette dernière décroît au début de l'évènement respiratoire et augmente au moment du micro-réveil qui suit l'évènement, ou bien présente une variabilité caractéristique pendant la survenue de l'évènement.

Pour améliorer le diagnostic, il est également possible de combiner l'analyse du PEA à une analyse d'un signal de pression nasale, par exemple recueilli grâce à un capteur de pression via une canule nasale, ou encore d'un signal d'un capteur de saturation sanguine en oxygène.

## Revendications

1. Un appareillage pour la détection de la survenue d'apnées ou d'hypopnées chez un patient, comprenant :
- des moyens de recueil de l'accélération endocardiaque (EA) du patient, et
- des moyens d'analyse, aptes à :
· déterminer au moins un paramètre fonction de l'un et/ou de l'autre de deux pics de l'accélération endocardiaque au cours d'un cycle donné, ces deux pics comprenant un premier pic (PEA I) lors de la phase de contraction ventriculaire isovolumique et un second pic (PEA II) lors de la phase de relaxation ventriculaire isovolumique,
· comparer ledit au moins un paramètre à un premier seuil prédéterminé (S), et
· délivrer conditionnellement un signal d'alerte (ALM) d'apnée ou d'hypopnée au franchissement de ce seuil,
appareillage **caractérisé en ce qu'**il est adapté à une détection non invasive de la survenue des apnées ou hypopnées, et **en ce que** :
- ledit capteur accélérométrique est un capteur accélérométrique externe (12) apte à être maintenu en contact avec la paroi thoracique du patient (10),
- ledit au moins un paramètre est fonction d'un ratio (R) entre une moyenne à long terme et une moyenne à court terme des valeurs de pic(s) d'accélération endocardiaque relevées au cours d'une pluralité de cycles successifs,
- ce ratio (R) est comparé audit premier seuil (S) pour délivrer ledit signal d'alerte (ALM) au franchissement de ce seuil, et
- les moyens d'analyse comprennent en outre des moyens aptes, après franchissement dudit premier seuil (S), à :
· détecter un franchissement inverse de ce premier seuil suivi du franchissement d'un second seuil (S'), supérieur au premier, et
· délivrer un signal de confirmation d'épisode d'apnée ou d'hypopnée au franchissement de ce second seuil (S').

2. L'appareillage de la revendication 1, dans lequel les moyens d'analyse sont aptes à délivrer ledit signal d'alerte après franchissement dudit premier seuil seulement si ce premier seuil reste franchi pendant une durée minimale prédéterminée, ou pendant un nombre minimal prédéterminé de cycles cardiaques.

3. L'appareillage de la revendication 1, dans lequel les moyens d'analyse sont aptes à délivrer ledit signal de confirmation d'épisode d'apnée ou d'hypopnée seulement si le franchissement dudit second seuil intervient au cours d'une durée maximale prédéterminée, ou pendant un nombre maximal prédéterminé de cycles cardiaques, après ledit franchissement inverse du premier seuil.

4. L'appareillage de la revendication 1, dans lequel les moyens d'analyse comprennent une machine à états ou réseau de neurones apte à comparer une pluralité desdits paramètres à une pluralité de seuils prédéterminés, à détecter le franchissement des différents seuils, à analyser la séquence de ces franchissements et à délivrer ledit signal d'alerte sur détection d'une ou plusieurs séquence(s) de franchissement prédéterminées.

5. L'appareillage de la revendication 1, dans lequel les moyens d'analyse comprennent des moyens aptes à appliquer à l'accélération endocardiaque un traitement d'autocorrélation, d'analyse morphologique, d'analyse fréquentielle, d'analyse par ondelettes et/ou d'analyse en composantes principales.

6. L'appareillage de la revendication 1, comprenant en outre des moyens (16) de recueil d'au moins une donnée physiologique parmi : la fréquence cardiaque, la pression nasale et/ou la saturation sanguine en oxygène du patient, et dans lequel les moyens d'analyse sont des moyens aptes à délivrer conditionnellement le signal d'alerte en fonction à la fois de ladite au moins une donnée physiologique et de la valeur prise par ledit au moins un paramètre.

7. L'appareillage de la revendication 1, dans lequel au moins un autre paramètre est fonction :
(i) d'une moyenne, et/ou
(ii) de la variation, et/ou
(iii) d'une différence, ou d'un ratio, entre une moyenne à long terme et une moyenne à court terme,
des valeurs de pic(s) d'accélération endocardiaque relevées au cours d'une pluralité de cycles successifs.

8. L'appareillage de la revendication 1, dans lequel au moins un autre paramètre est fonction de l'intervalle entre complexe QRS et au moins l'un des pics d'accélération endocardiaque, et/ou de l'intervalle entre le premier et le second pic d'accélération endocardiaque.

## Claims

1. A device for detecting the occurrence of apneas or hypoapneas in a patient, comprising :
- means for acquiring the endocardial acceleration (EA) of a patient, and
- analyzing means, adapted for determining at least one parameter that is a function of the one and/or the other of two peak endocardial acceleration values during a given cycle, said two peaks comprising a first peak (PEA I) during the isovolumic ventricular contraction phase and a second peak (PEA II) during the isovolumic ventricular relaxation phase,
- comparing said at least one parameter with a first predetermined threshold (S), and
- conditionally deliver an apnea or hypoapnea alarm signal (ALM) when passing said threshold,
wherein said device is **characterized in that** it is adapted to a non-invasive detection of the occurrence of apneas or hypoapneas and **in that** :
- said acceleration sensor is an external acceleration sensor (12) that can be maintained in contact with patient's chest wall (10),
- said at least one parameter is a function of a ratio (R) between a long term average and a short term average of the peak endocardial acceleration value(s) observed during a plurality of successive cycles,
- wherein said ratio (R) is compared with said first threshold (S) for delivering said alarm signal (ALM) when passing said threshold, and
- the analyzing means moreover comprise means adapted for
- detecting a reverse passing of said first threshold followed by the passing of a second threshold (S') that is greater than the first one, and
- delivering a signal for confirming the apnea or hypoapnea episode when passing said second threshold (S')
after passing said first threshold (S').

2. The device according to Claim 1, wherein the analyzing means are adapted for delivering said alarm signal after passing said first threshold only if said first threshold continues to be passed during a predetermined minimum duration or during a predetermined minimum number of cardiac cycles.

3. The device according to Claim 1, wherein the analyzing means are adapted for delivering said confirmation signal of apnea or hypoapnea episode only if the passing of said second threshold occurs during a predetermined maximum duration or during a predetermined maximum number of cardiac cycles, after said reverse passing of the first threshold.

4. The device according to Claim 1, wherein the analyzing means comprise a state machine or a neuronal network adapted for comparing a plurality of said parameters with a plurality of predetermined thresholds, detecting the passing of the different thresholds, analyzing the sequence of these passings and delivering said alarm signal when detecting one or more sequence (s) of predetermined passings.

5. The device according to Claim 1, wherein the analyzing means comprise means adapted for applying to the endocardial acceleration value an autocorrelation treatment, a morphological analysis, a frequency analysis, a wavelet analysis and/or a principal component analysis.

6. The device according to Claim 1, moreover comprising means (16) for acquiring at least one physiological data selected among heart rate, nasal pressure and/or oxygen saturation of patient's blood, and wherein analyzing means are means adapted to conditionally deliver the alarm signal as a function both of said at least one physiological date and of the value taken by said at least one parameter.

7. The device according to Claim 1, wherein at least one further parameter is a function
(i) of an average value, and/or
(ii) of the variation, and/or
(iii) of a difference or a ratio between a long term average and a short term average
of the values of the peak endocardial acceleration(s) acquired during a plurality of successive cycles.

8. The device according to Claim 1, wherein at least one further parameter is a function of the interval between the QRS complex and at least one of the peak endocardial acceleration values and/or of the interval between the first and the second peaks of endocardial acceleration.

## Patentansprüche

1. Eine Vorrichtung zum Nachweis des Auftretens von Apnoen bzw. Hypoapnoen bei einem Patienten, umfassend :
- Mittel zur Erfassung der endokardialen Beschleunigung (EA) des Patienten, und
- Analysemittel, die geeignet sind,
- wenigstens einen Parameter zu bestimmen, der von einem und/oder dem anderen der zwei Spitzenwerte eines gegebenen Zyklus abhängt, wobei die besagten zwei Spitzenwerte einen ersten Spitzenwert (PEA I) während der isovolumischen Ventrikelkontraktionsphase und einen zweiten Spitzenwert (PEA II) während der isovolumischen Ventrikelrelaxationsphase umfassen,
- den wenigstens einen Parameter mit einer ersten vorbestimmten Schwelle (S) zu vergleichen, und
- bedingt ein Apnoe- bzw. Hypoapnoe-Alarmsignal beim Überschreiten dieser Schwelle zu liefern,
wobei die Vorrichtung einem nichtinvasiven Nachweis des Auftretens von Apnoen bzw. Hypoapnoen angepasst ist und **dadurch gekennzeichnet ist, dass** :
- der besagte Beschleunigungssensor ein externer Beschleunigungssensor ist, der geeignet ist, den Kontakt mit der Brustwand des Patienten (10) beizubehalten,
- der besagte wenigstens ein Parameter von einem Verhältnis (R) zwischen einem Langzeitmittelwert und einem Kurzzeitmittelwert des bzw. der während einer Vielzahl aufeinanderfolgender Zyklen gemessenen Spitzenwerte der endokardialen Beschleunigung abhängt,
- dieses Verhältnis (R) mit der besagten ersten Schwelle (S) verglichen wird, um das besagte Alarmsignal (ALM) beim Überschreiten dieser Schwelle zu liefern, und
- die Analysemittel ausserdem Mittel aufweisen, die nach dem Überschreiten der besagten ersten Schwelle (S) geeignet sind, :
- ein umgekehrtes Überschreiten dieser ersten Schwelle gefolgt vom Überschreiten einer Schwelle (S'), die grösser als die erste ist, nachzuweisen,
- ein Signal zur Bestätigung einer Apnoe- bzw. Hypoapnoe-Episode beim Überschreiten jener zweiten Schwelle (S') zu liefern.

2. Die Vorrichtung nach Anspruch 1, in welcher die Analysemittel geeignet sind, das besagte Alarmsignal nach dem Überschreiten der ersten Schwelle zu liefern, erst wenn diese erste Schwelle während einer vorbestimmten Dauer oder während einer vorbestimmten minimalen Zahl von Herzzyklen überschritten bleibt.

3. Die Vorrichtung nach Anspruch 1, in welcher die Analysemittel geeignet sind, das besagte Signal zur Bestätigung einer Apnoe- bzw. Hypoapnoe-Episode zu liefern, erst wenn das Überschreiten der besagten zweiten Schwelle während einer vorbestimmten maximalen Dauer oder während einer vorbestimmten maximalen Zahl von Herzzyklen nach dem umgekehrten Überschreiten der ersten Schwelle auftritt.

4. Die Vorrichtung nach Anspruch 1, in welcher die Analysemittel einen endlichen Automat oder ein Neuronnetzwerk umfassen, und die geeignet ist, eine Vielzahl von besagten Parametern mit einer Vielzahl von vorbestimmten Schwellen zu vergleichen, das Überschreiten der verschiedenen Schwellen nachzuweisen, die Sequenz dieser Überschreiten zu analysieren und das besagte Signal beim Nachweis einer oder mehrerer vorbestimmten Überschreitungssequenz(en) zu liefern.

5. Die Vorrichtung nach Anspruch 1, in welcher die Analysemittel Mittel aufweisen, die dazu geeignet sind, die endokardiale Beschleunigung durch Autokorrelation, morphologische Analyse, Frequenzanalyse, Wellchenanalyse und/oder Hauptkomponentenanalyse zu behandeln.

6. Die Vorrichtung nach Anspruch 1, die ausserdem Mittel (16) zur Erfassung wenigstens eines physiologischen Messwerts ausgewählt unter der Herzfrequenz, dem Nasendruck und/oder der Blutsauerstoffsättigung umfasst und in welcher die Analysemittel Mittel sind, die geeignet sind, bedingt das Alarmsignal in Abhängigkeit sowohl von jenem wenigstens einen physiologischen Messwert als auch vom Wert des besagten wenigstens einen Parameters zu liefern.

7. Die Vorrichtung nach Anspruch 1, in welcher wenigstens ein anderer Parameter
(i) von einem Mittelwert, und/oder
(ii) von der Veränderung, und/oder
(iii) von einem Unterschied oder einem Verhältnis zwischen einem Langzeitmittelwert und einem Kurzzeitmittelwert,
von den während einer Vielzahl von aufeinanderfolgenden Zyklen gemessenen Spitzenwerten der endokardialen Beschleunigung(en), abhängig ist.

8. Die Vorrichtung nach Anspruch 1, in welcher wenigstens ein anderer Parameter vom Intervall zwischen QRS-Komplex und wenigstens einem der Spitzenwerte der endokardialen Beschleunigung und/oder vom Intervall zwischen dem ersten und dem zweiten Spitzenwerte der endokardialen Beschleunigung abhängig ist.
